(19)

Europäisches
Patentamt
European
Patent Office
Office européen
des brevets

(11) **EP 4 006 136 A1**

(12) **EUROPEAN PATENT APPLICATION**
published in accordance with Art. 153(4) EPC

(43) Date of publication:
**01.06.2022 Bulletin 2022/22**

(21) Application number: **20844677.3**

(22) Date of filing: **17.07.2020**

(51) International Patent Classification (IPC):
**C12M 1/12** (2006.01)   **C12M 1/00** (2006.01)
**C12N 5/09** (2010.01)

(52) Cooperative Patent Classification (CPC):
**C12M 1/00; C12M 1/12; C12N 5/00**

(86) International application number:
**PCT/CN2020/102564**

(87) International publication number:
**WO 2021/013066 (28.01.2021 Gazette 2021/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.07.2019 CN 201910666239**

(71) Applicant: **Peking University**
**Beijing 100871 (CN)**

(72) Inventors:
• **YANG, Gen**
  **Beijing 100871 (CN)**

• **LUO, Chunxiong**
  **Beijing 100871 (CN)**
• **LU, Chunyang**
  **Beijing 100871 (CN)**
• **XU, Jian**
  **Beijing 100871 (CN)**
• **WANG, Yugang**
  **Beijing 100871 (CN)**
• **OUYANG, Qi**
  **Beijing 100871 (CN)**

(74) Representative: **Schnappauf, Georg**
**ZSP Patentanwälte PartG mbB**
**Hansastraße 32**
**80686 München (DE)**

(54) **MICROFLUIDIC CHIP SUITABLE FOR CAPTURING CIRCULATING TUMOUR CELLS**

(57)     A microfluidic chip capable of being used for capturing target particles, the chip comprising a convergence and shunt unit, the convergence and shunt unit being capable of converging target particles in a liquid sample to the centre of a liquid flow, and simultaneously splitting off a certain proportion of the liquid flow that does not contain target particles, thereby effectively reducing the flow and speed of the liquid flow inputted to the chip; when used for capturing target particles, the chip also comprises a capturing unit, and implements capture of the target particles by means of the capturing unit.

**FIG. 2**

EP 4 006 136 A1

**Description**

**CROSS REFERENCE TO RELATED APPLICATIONS**

This application claims the priority of Chinese Patent Application No. 201910666239.5, filed with the China National Intellectual Property Administration on July 23, 2019.

**FIELD**

The present disclosure relates to the field of liquid biopsy and tumor therapy through physical method, and specifically relates to a microfluidic chip for capturing tumor cells (especially CTCs).

**BACKGROUND**

Blood contains a huge number of red blood cells (about $5 \times 10^9$ cells/mL), white blood cells (about $8 \times 10^6$ cells/mL) and various proteins, so it has strong granularity and high dynamic viscosity coefficient. Recent studies have shown that cancer patients, especially those with metastatic cancer lesions, have a certain number of circulating tumor cells (CTCs), and the successful capture and separation of these circulating tumor cells is of inestimable value for the early detection, diagnosis and treatment of cancer. However, due to the extremely low number of CTCs in the blood (about 0 to tens of cells/mL), there are great technical difficulties on capturing circulating tumor cells efficiently from blood.

At present, based on the different principles of capturing CTCs, methods for separating CTCs can be divided into three categories: capture method based on antigen and antibody, capture method based on physical properties, and capture method based on the combination of both.

Capture method based on antigen and antibody

Some tumor-specific antigens, such as EpCAM, CEA and HER2, are expressed on the surface of tumor cells. Antibodies that recognize tumor-specific antigens are fixed on the surface of magnetic beads or microfluidic chips via chemical bonds or other coating methods, and tumor cells are captured by the binding between antigen and antibody.

CellSearch is currently the only platform approved by the FDA for commercial use for the separation of CTCs, but its detection rate is not high. Subsequently, some methods combine the antigen-antibody specific binding with microfluidic chip, which greatly improves the detection rate. For example, HB-chip employs asymmetrically arranged herringbone channels to increase the Reynolds number. As a result, after the blood of cancer patients flows into the chip, turbulence can be formed, thereby increasing the interaction between cells and the wall of channel, which improves the probability of the binding between cells and antibodies, and the detection rate is significantly improved compared with the CellSearch method. In addition, CTC-chip adopts staggered cylindrical surface coated with EpCAM antibody to capture CTCs.

The advantages of capturing CTC based on antigen and antibody binding are obvious. Due to the high specificity of antibody for antigen recognition, the purity of the captured cells is very high, almost close to 100%. However, this method also has many disadvantages. For example: (1) The specificity and the affinity between antigen and antibody limit the capture efficiency, because the molecular marker currently used is mainly EpCAM, but some CTCs have undergone EMT, and these cells with low EpCAM expression will be missed, resulting in an underestimation of the number of CTCs; (2) The cost is relatively high; (3) Since the antigen and antibody need time to contact and bind, the flow rate is generally slow and the processing time is long, which affects blood properties and CTC status. The existence of the above shortcomings restricts its large-scale clinical application.

Capture method based on physical properties

Studies have shown that there are certain differences in physical properties between tumor cells and normal cells, which are mainly reflected in cell size, deformability and density. Therefore, using the above-mentioned differences in physical properties, CTCs can be separated and captured. In the prior art, capture based on physical properties often involves a spiral structure. On account of the small characteristic size and low flow rate in the microchannel, the Reynolds number is generally less than 1, and the inertial effect is usually ignored. However, driven by pressure, a very high flow rate of 0.1-1 m/s and the Reynolds number of 10-100 can be reached, where the inertial effect begins to manifest, and the spiral structure takes advantage of the above characteristics. In such a chip, the diluted blood is pushed into the curved channel by pressure. Under the action of lateral force and Dean force, cells will reach a specific equilibrium position in the cross section. As the equilibrium position of cells is determined by the cell size, and the size of the CTCs is quite different from the size of the blood cells, CTCs will be in different equilibrium position in the channel and separated.

Besides, there are many other separation methods based on physical properties, for example, by using suitable gaps, separation is achieved based on the difference in size and deformability between CTCs and blood cells.

The advantages of capturing CTC based on physical properties are as follows: (1) The capture is independent of antigen expression, and can capture all types of CTCs; (2) CTCs do not bind to antibodies or nanoparticles, maintaining the most primitive state; (3) The blood sample require no pretreatment and can be tested directly; (4) The cost is less; (5) The flow rate is high and the time required is short. However, since the sizes of CTCs and white blood cells are slightly overlap, the separation efficiency of this method is often not high enough, and there is a problem of white blood cell contamination.

Capture method based on the combination of both

Using a combination of antigen-antibody-based methods and physical properties-based methods, Sun N et al. utilized antibody-coated magnetic beads to bind to CTCs, which effectively increased the size of CTCs, and then conducted filtration with porous membranes, which significantly improved the capture efficiency, while using CD45 negative selection to remove white blood cells, effectively improved the purity. CTC-ichip uses DLD to separate large cells from small ones, which are then collected, and immunomagnetic beads are used to remove white blood cells by negative selection. However, these methods often need complicated chip structures, cumbersome process and high cost, which is not suitable for clinical application.

There is an urgent need in the art for an effective CTC capture method that can meet the requirements for low cost, high sample throughput and high accuracy in clinical applications.

**SUMMARY**

After years of research, the inventors invent a microfluidic chip that can be used to capture target particles (such as circulating tumor cell (CTC)). The chip comprises a focusing-separating unit that can focus target particles in a liquid sample (such as blood, perfusion fluid, etc.) to the center of the liquid flow, and simultaneously separate away a certain proportion of the liquid flow that does not contain the target particles, thereby effectively reducing the flow velocity and/or flow discharge of the liquid sample input to the chip, making it more suitable for capturing target particles therein. When the chip is used for capturing target particles, it further comprises a capturing unit, through which the capturing of target particles is achieved. Specifically, the present disclosure comprises the following embodiments.

In the first aspect, the present disclosure provides a microfluidic chip comprising an inlet (1), a main channel (2), one or more (for example, 1-20, preferably 1, 2, 3, 4, 5, 6, 7, 8, 9 or 10) of focusing-separating units (3) and an outlet (5), wherein the inlet (1), the focusing-separating unit (3) and the outlet (5) are connected by the main channel (2);

when a liquid sample enters the main channel (2) from the inlet (1) and flows through the focusing-separating unit (3), the focusing-separating unit (3) focusses target particles in the sample to the center of the liquid flow, and simultaneously discharges or partially discharges the liquid flow that does not contain the target particles from the outlet (5), thereby reducing the flow velocity and/or flow discharge of the sample without losing the target particles.

In one embodiment, the focusing-separating unit (3) comprises a collecting port (31), a narrow segment (32) of the main channel, one, two or more (for example, 1-20, such as 1, 2, 3, 4, 5, 6, 7, 8, 9, 10) of series-arranged focusing structure (33), and a separating channel (34).

In another embodiment, the focusing structure (33) comprises a central channel (331) and a branch channel (332), wherein the central channel (331) is connected to the main channel (2) at both ends and arranged coaxially; preferably, the width of the central channel (331) is smaller than the width of the main channel (2), also preferably, the width of the central channel (331) is 30%-99% of the width of the main channel (2), such as 95%, 90%, 85%, 80%, 75%, 70%, 65%, 60%, 55%, 50%, 45%, 40%, 35%, 30%, or a value between any two above; and the branch channel (332) intersects the main channel (2) and the central channel (331) at both ends; further preferably, the two branch channels (332) are arranged at both sides of the central channel (331), more preferably, the two branch channels (332) are arranged symmetrically at both sides of the central channel (331), and share the same size parameter.

In yet another embodiment, the flow resistance of the central channel (331) has a proportional relationship with the flow resistance of the branch channel (332), so that when the sample enters the focusing structure (33) from the main channel (2), the liquid flow containing the target particles enters the central channel (331), while the liquid flow containing no target particles flows into the branch channel (332), then merges with the liquid flow from the central channel (331), and then flows into the main channel (2) again.

The term "flow resistance" is a reaction force that hinders the flow when a liquid (viscous liquid) moves in a certain space (such as a channel). For the liquid flowing in the channel, the flow resistance $R_{flow\ resistance}$ is expressed as: $R_{flow\ resistance} = 8\eta L(H+W)^2/(HW)^3$, where $\eta$ represents the viscosity coefficient of the liquid, and L, W and H respectively represent the length, width, and height of the channel. The flow resistance of a specific channel in the present disclosure (such as the flow resistance of the central channel, the flow resistance of the branch channel, etc.) refers to the resistance generated when the liquid flows in the indicated channel. For different channels, the liquid flow is the same and has the same viscosity coefficient. Therefore, the proportional relationship of the flow resistance generated in different channels for the liquid is only related to the size of the channel, and can be derived based on the ratio of $L(H+W)^2/(HW)^3$ of the channels. It should be noted that the above-mentioned flow resistance expression and the calculation of the proportional relationship of flow resistance are not limited to the central channel (331) and the branch channel (332), but are applicable to all channels in the microfluidic chip.

According to the Poiseuille formula $Q=\Delta p/R$, where Q represents the flow rate of the liquid flow, $\Delta p$ represents the pressure difference between the two ends of the channel, and R represents the flow resistance of the channel. It can be concluded that the flow Q is inversely proportional to the flow resistance R, that is, Q1/Q2=R2/R1.

In yet another embodiment, when $W_Z$ represents half the width of the main channel (2), $W_2$ represents the width of the narrow segment (32) of the main channel, $r_{cell}$ represents the average radius of the target particles in the sample, d1 represents the distance between the centroid of the target particles closest to one side boundary (for example, the right side, similarly, also applicable to the left side) of the narrow segment (32) and the close side boundary, R1 is the flow resistance of the central channel (331) of the first focusing structure (33) in each focusing-separating unit (3), and R2 is the flow resistance of a single branch channel (332) in the first focusing structure (33), the following formula is met:

$$\frac{3}{2}\frac{W_2-2d1}{W_2} - \frac{1}{2}\left(\frac{W_2-2d1}{W_2}\right)^3 \leq \frac{R2}{R2+2R1} \qquad (VIII)$$

Wherein d1 is equal to $r_{cell}$, or slightly smaller than $r_{cell}$. The left side of the inequality represents the volume ratio of the liquid flow remaining in the main channel to the overall liquid flow in the main channel, and the right side represents the ratio of overall flow resistance to the flow resistance of the central channel in the focusing structure.

If the above formula (inequality) is satisfied, it can be ensured that the first focusing structure would not allow the target particles leaving, and meanwhile as far as possible to split out the fluid without target particles. Further, the proportional relationship that needs to be satisfied between the flow resistance of the central channel (331) of the focusing structure and the flow resistance of the branch channel (332) can be calculated.

In yet another embodiment, when the flow resistance of the central channel (331) of the $n^{th}$ (n is a natural number greater than or equal to 1 and less than the total number of the focusing structures) focusing structure (33) is $R1^n$, the flow resistance of a single branch channel (332) is $R2^n$, the flow resistance of the central channel (331) of the $(n+1)^{th}$ focusing structure (33) is $R1^{n+1}$, and the flow resistance of each branch channel (332) is $R2^{n+1}$, the following formula is met:

$$\frac{R2^n}{R2^n+2R1^n}\left(\frac{3}{2}\frac{W_s{}^n-2d1}{W_s{}^n} - \frac{1}{2}\frac{(W_s{}^n-2d1)^3}{(W_s{}^n)^3}\right) \leq \frac{R2^{n+1}}{R2^{n+1}+2R1^{n+1}} \qquad (IX)$$

Wherein d1 is equal to $r_{cell}$, or slightly smaller than $r_{cell}$. The left side of the inequality represents the volume ratio of the liquid flow containing the target particles (the liquid flow remaining in the main channel) after flowing through the $n^{th}$ focusing structure to the overall liquid flow in the main channel, where $\dfrac{R2^n}{R2^n+2R1^n}$ represents the ratio of the flow rate of the central channel of the $n^{th}$ focusing structure to the overall flow rate, and

$$\left(\frac{3}{2}\frac{W_s{}^n-2d1}{W_s{}^n} - \frac{1}{2}\frac{(W_s{}^n-2d1)^3}{(W_s{}^n)^3}\right)$$

represents the ratio of the flow rate of the liquid containing the concentrated target particles to the flow rate of the whole central channel, and the right side represents the overall flow resistance of the $(n+1)^{th}$ focusing structure to the flow resistance of the central channel of the focusing structure.

If the above formula (inequality) is satisfied, it can be ensured that when flowing through the $n^{th}$ focusing structure, that is, when entering the $(n+1)^{th}$ focusing structure, the target particles will not leave the main channel, and meanwhile the fluid containing no target particles will split out as much as possible. Further, the proportional relationship that needs to be satisfied between the flow resistances of the $n^{th}$ and the $(n+1)^{th}$ focusing structures can be calculated.

In yet another embodiment, the separating channel (34) intersects with the main channel (2) at both ends, and upstream intersection is close to the last focusing structure in the focusing-separating unit, and downstream intersection is close to the outlet (5) of the microfluidic chip; preferably, there are two separating channels (34) arranged at both sides of the main channel (2); more preferably, the two separating channels (34) are arranged symmetrically at both sides of the main channel (2) and share the same size parameter; also preferably, at the intersection of the separating channel (34) and the main channel (2), the width of the main channel becomes larger, for example, the width is 1.5-5 times of the original width, such as 1.5 times, 2 times, 2.5 times, 3 times, 3.5 times, 4 times, 4.5 times or 5 times, or a value between any two above; further preferably, the wall of the channel at the intersection is round.

In yet another embodiment, the flow resistance of the separating channel (34) has a proportional relationship with the overall flow resistance of the area between the two intersections of the separating channel (34) and the main channel (2), so that the target particles continue to flow into the main channel (2), while the liquid flow that does not contain the target particles flows into the separating channel (34).

In yet another embodiment, the flow resistance of the separating channel (34) of the $m^{th}$ (m is a natural number greater than or equal to 1, and less than or equal to the total number of the focusing-separating units, such as m = 1, 2, 3, 4, 5, 6 , 7, 8, 9 or 10) focusing-separating unit (3) is determined as follows: after the sample flows through the last focusing structure (33) of the $m^{th}$ focusing-separating unit (3), the target particles are concentrated in the range of [-r, r] from the center of the main channel (2), where r is calculated based on the ratio of the channel width to the flow resistance of each focusing structure in the $m^{th}$ focusing-separating unit; if half the width of the main channel is $W_Z$, the width of the liquid separated by the separating channel (34) is less than or equal to $W_Z$-r, and then the ratio of the liquid flow in the separating channel (34) to the overall liquid flow is calculated as

$$k = 1 - \frac{3}{2}\frac{r}{W_Z} + \frac{1}{2}\frac{r^3}{W_Z{}^3};$$

if the overall flow resistance of the area between the two intersections of the separating channel (34) and the main channel (2) of the $m^{th}$ focusing-separating unit (34) (or the overall flow resistance of the internal area of the separating channel) is $R1^m$, and the flow resistance of each separating channel (34) is $R2^m$, according to the formula:

$$\frac{k}{1-k} = \frac{R1^m}{\frac{R2^m \cdot R2^m}{R2^m + R2^m}} = \frac{2R1^m}{R2^m}$$

is obtained, and the proportional relationship between $R2^m$ and $R1^m$ is obtained as

$$R2^m = \frac{2(1-k)}{k} R1^m,$$

that is the relationship between the flow resistance of the separating channel of the $m^{th}$ focusing-separating unit and the flow resistance of all subsequent structures. From back to front, based on the overall flow resistance of the last separating channel, i.e., the flow resistance of the capture unit, any one of the previous ones, such as the flow resistance of the separating channel (34) of the $m^{th}$ focusing-separating unit (3) is obtained, and further, the specific size or size range of the channel can be obtained.

In yet another embodiment, the microfluidic chip further comprises a capturing unit (4) for capturing target particles; preferably, the capturing unit (4) is located downstream of the focusing-separating unit (3), and both ends are connected to the main channel (2); also preferably, the capturing unit is connected to the main channel (2) through a branch channel (41).

In a particular embodiment, the capturing unit (4) comprises one layer or more than one layer (for example, 1, 2, 3, 4, 5, 6, 7, 8, 9 or 10 layers) of array, preferably, the array is formed by arranging small blocks (42) of any shape (such as cube, rectangular parallelopiped, triangular prism, cylinder, etc., or with a cross section of square, rectangle, triangle, circle, etc.), and there is a gap d between adjacent small blocks (42), wherein the gap d is defined as the closest distance between the surfaces of two adjacent small blocks (42); also preferably, the gap d between the small blocks (42) in each layer of the array is identical; further preferably, the gap d between the small blocks (42) in different layers of the array is gradually decreased from top to bottom, for example, when the number of layers of the array is 4, the gap d from top to bottom is 14 μm, 12 μm, 10 μm and 8 μm.

In yet another embodiment, the inlet (1) further comprises a filtering structure (11).

In the second aspect, the present disclosure provides a device for enriching and/or capturing target particles in a sample, comprising the microfluidic chip in the first aspect.

In the third aspect, the present disclosure provides a method for reducing the flow discharge and/or flow velocity of a sample, comprising using the microfluidic chip in the first aspect or the device in the second aspect.

In the fourth aspect, the present disclosure provides a method for enriching and/or capturing target particles, comprising:

(1) providing a liquid sample containing target particles; and

(2) injecting the liquid sample into the microfluidic chip in the first aspect or the device in the second aspect.

In a fifth aspect, the present disclosure provides a method for removing target particles in a liquid sample, comprising:

(1) providing a liquid sample containing target particles;

(2) injecting the liquid sample into the microfluidic chip in the first aspect or the device in the second aspect; and

(3) collecting the liquid sample flowing out from the outlet (5) of the microfluidic chip.

Optionally, repeating steps (2) and (3), and the repeating is performed 1, 2, or more times (for example, 3, 4, 5, 6, 7, 8, 9 or 10 times).

In the method of the present disclosure, the liquid sample is injected at a rate of 5-200 mL/h, 10-150 mL/h, 20-100 mL/h, 30-80 mL/h, 40-60 mL/h, and specifically, the rate is 10, 20, 30, 40, 50, 60, 70, 80, 90, 100, 110, 120, 130, 140, 150, 160, 170, 180, 190 or 200 mL/h, or a value between any two above.

The sample used in all embodiments of the present disclosure is whole blood, plasma, serum, perfusion fluid, urine, tissue fluid, cerebrospinal fluid, cell culture fluid or cell mixture, preferably whole blood or perfusion fluid.

The target particles involved in all embodiments of the present disclosure are tumor cells, preferably circulating tumor cells (CTC).

The chip of the present disclosure realizes the capture of tumor cells in the blood at a high flow rate of at least 40 mL/h with a capture efficiency of greater than 90% and an average residual rate of white blood cells of only 0.008%, which is much lower than other physical separation methods. Since the chip of the present disclosure is suitable for the separation and capture of target particles (cells) in high-flow rate samples, clinical applications become possible. Moreover, it has been used successfully in double-blind negative and positive identification and separation of blood samples from healthy volunteers and patients with diagnosed lung cancer, breast cancer, liver cancer, etc.

**BRIEF DESCRIPTION OF DRAWINGS**

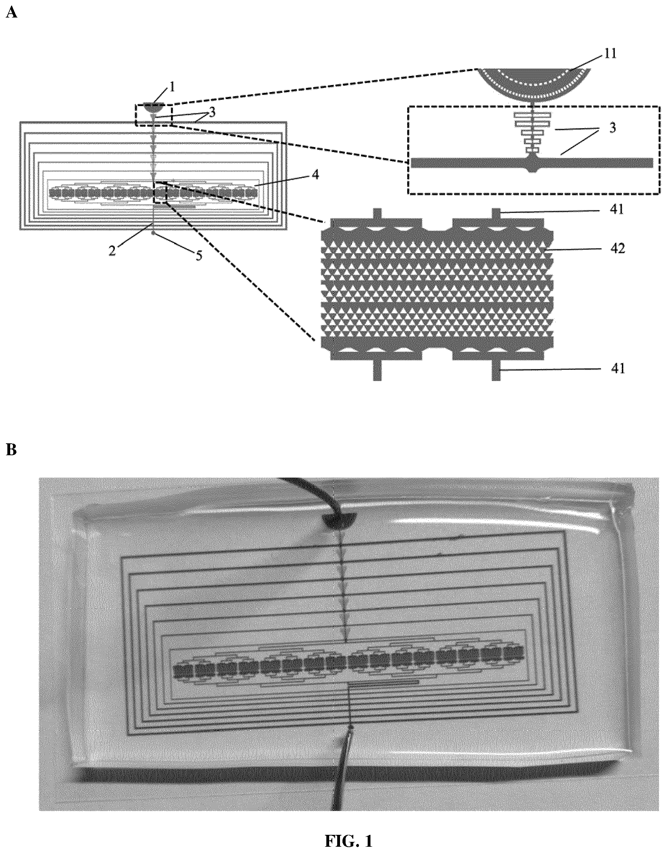

FIG. 1 shows the schematic diagram (A) and physical image (B) of the chip of Example 1.

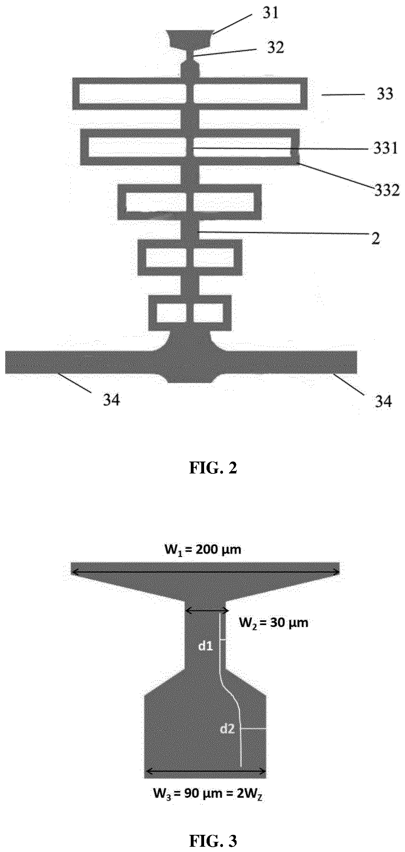

FIG. 2 shows a schematic diagram of a focusing-separating unit in one embodiment, which has five focusing structures and a separating channel on both sides of the main channel.

FIG. 3 shows a schematic diagram of a narrow segment of the main channel in one embodiment, wherein d1 represents the distance between the centroid of the target particles and the boundary of the narrow segment of the main channel, and d2 represents the distance between the centroid of the target particles and the boundary of the main channel.

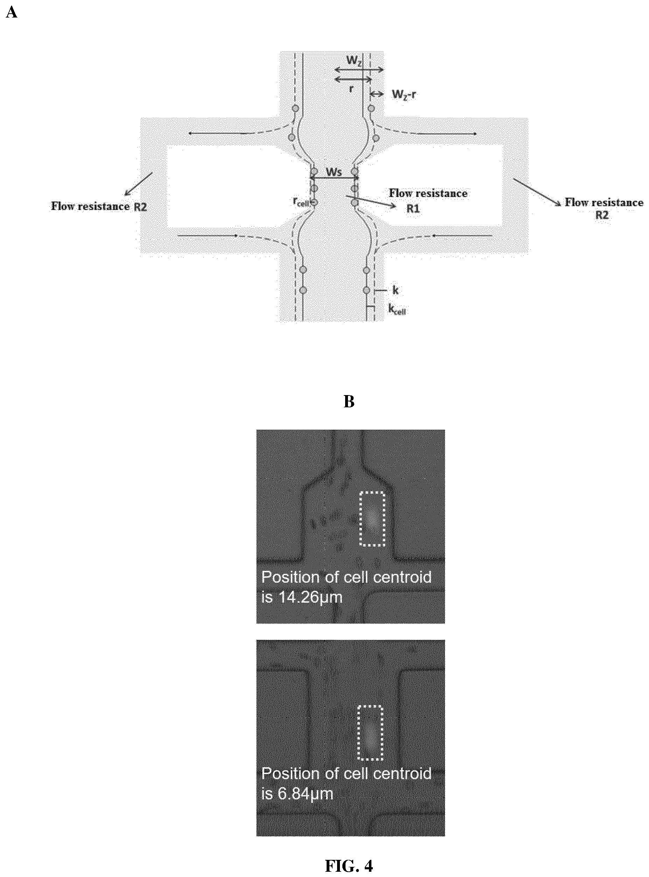

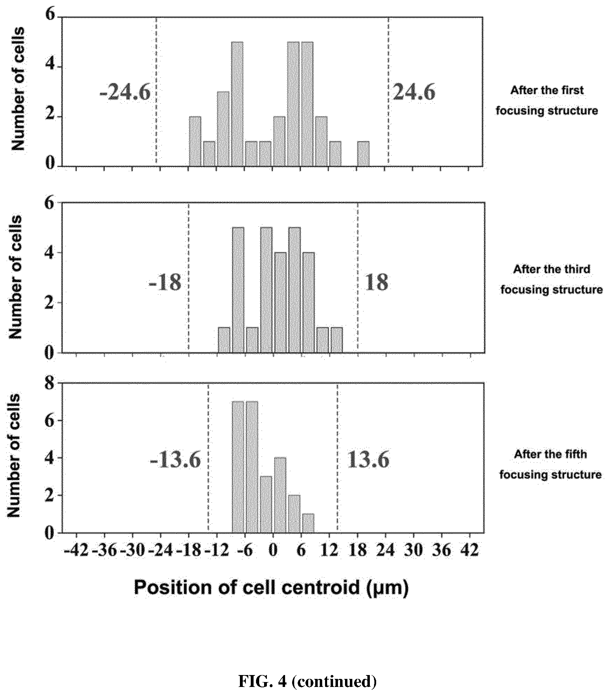

FIG. 4A shows a streamline diagram of the focusing structure, wherein r represents the distance between the boundary (close to the center side) of the liquid flow (which will split out) and the center of the main channel; Wz represents half the width of the main channel. FIG. 4B shows the position of fluorescence labeled tumor cells (distance between cell centroid and the center of main channel) in the main channel after flowing through the first focusing structure in one embodiment. FIG. 4C shows the position distribution of the tumor cells in the main channel

after flowing through the first, third, and fifth focusing structures in one embodiment.

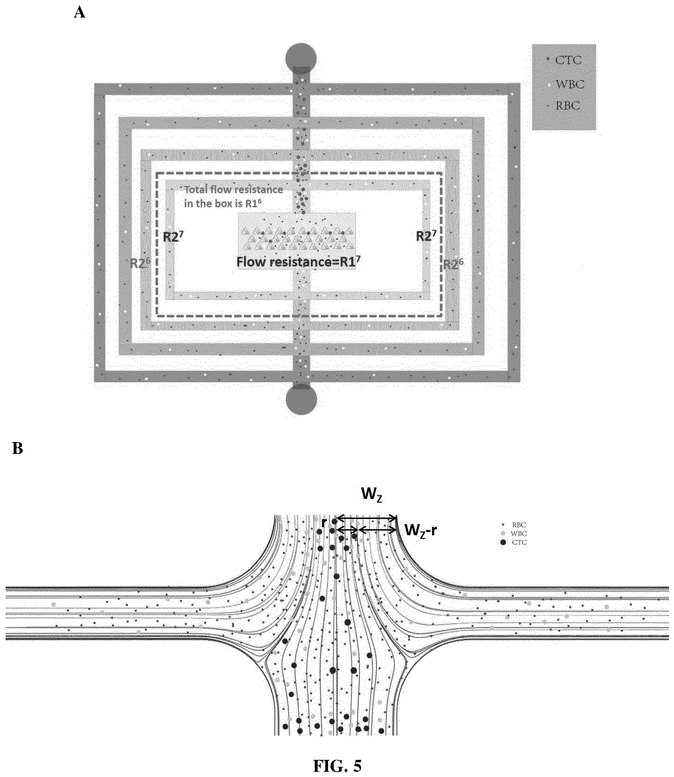

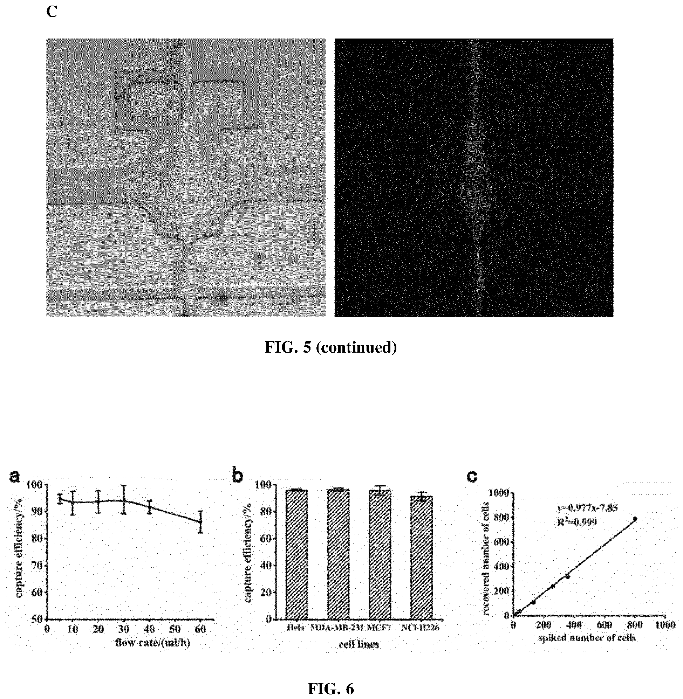

FIG. 5A shows the overall design of the separating channel in one embodiment. FIG. 5B shows a partial enlarged view of the separating channel. FIG. 5C shows the liquid flow streamline of the blood sample containing fluorescence labeled tumor cells when passing the separating channel, where the left panel is a merged image of bright field and fluorescence field, and the right panel is a fluorescence image.

FIG. 6A shows the capture efficiency on HeLa cells mixed in blood at different flow rates. FIG. 6B shows the capture efficiency on tumor cells of different cell lines mixed in blood at a flow rate of 40 mL/h. FIG. 6C shows the capture efficiency on HeLa cells with different concentrations mixed in blood at a flow rate of 40 mL/h.

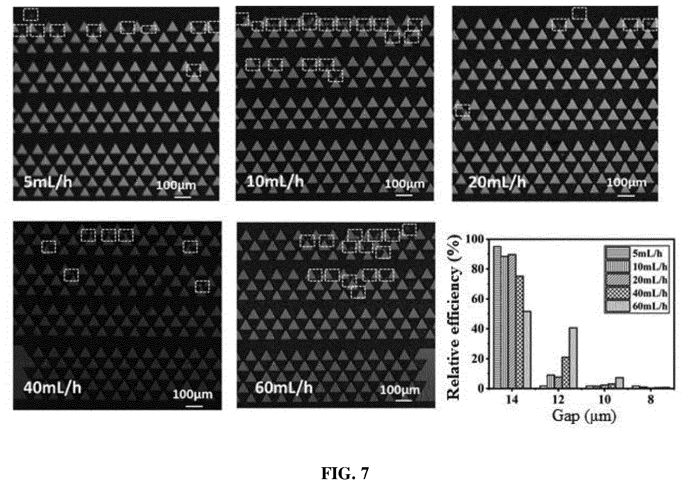

FIG. 7 shows the distribution of tumor cells (mixed in blood) in the capture area at different flow rates, where the tumor cells are marked with white dashed boxes.

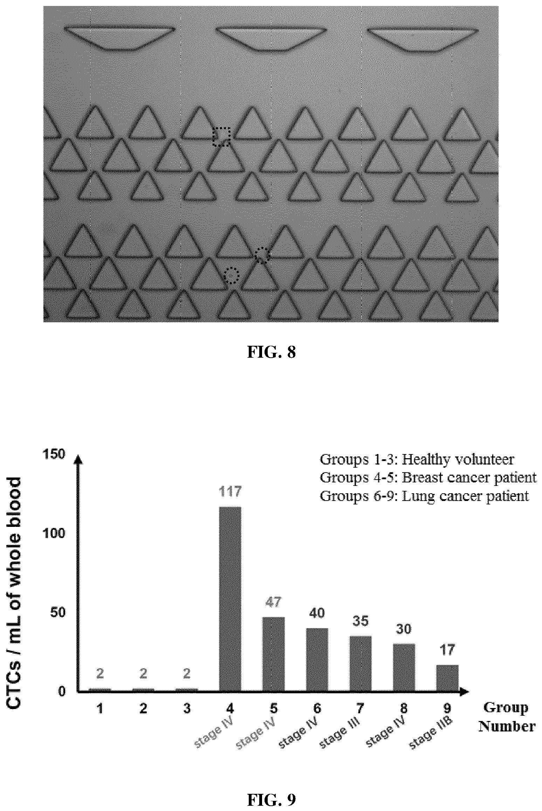

FIG. 8 shows a typical capture situation, wherein the blood sample with tumor cells flows through the chip and are washed with PBS, and red blood cells are broken with ACK lysis buffer. Tumor cells are stained with red fluorescence and indicated by a black dashed box in the figure, while white blood cells are stained with blue fluorescence and indicated by a black dashed circle in the figure.

FIG. 9 shows the results of a clinical double-blind test to detect the number of CTCs in the blood of healthy volunteers and tumor patients.

## DETAILED DESCRIPTION

The present invention can be further illustrated through examples.

Microfluidic Chip

The first function of the microfluidic chip of the present invention is to focus target particles to the central of the sample liquid flow and realize the reduction of the flow velocity and/or flow discharge of the liquid flow, so that facilitates subsequent capture or separation of target particles. In order to achieve this function, the chip of the present invention comprises an inlet (1), a main channel (2), a focusing-separating unit (3), and an outlet (5). To simultaneously capture the target particles, it preferably further comprises a target particle capturing unit (abbreviated as capturing unit) (4).

The function of the focusing-separating unit (3) is to focus the target particles (such as tumor cells) in the sample input into the chip to the center line of the main channel (2), while non-target particles (such as red blood cells, white blood cells, etc.) are separated, so that the relative concentration of target particles can be significantly increased. More importantly, when the sample flows through the focusing-separating unit and enters the capture unit 4, the flow velocity and flow discharge of the sample have been significantly reduced compared to that of before entering the focusing-separating unit (3), which also provides convenient conditions for the capture of target particles. This feature makes the chip of the present invention suitable for high-flow and high-throughput sample addition, which greatly improves the effect of actual clinical applications.

Inlet

Inlet (1) is the inlet for sample in the chip, which is used to input the sample to be tested into the microfluidic chip. In one embodiment, the inlet also comprises a filtering structure (11), so as to filter out impurities in the sample and prevent blockage in the chip.

Main channel

Main channel (2) is the main sample channel connecting inlet (1) and outlet (5) of the chip, and it has a cross-sectional width of $2W_z$ ($W_z$ is half of the cross-sectional width). Different functional modules (such as the focusing-separating unit (3)) are connected to the main channel for different types of operations on the samples. It should be noted that the main channel (2) is not a physically absolutely continuous channel, and the extension direction of the main channel (2) may have other structures. For example, a segment of the main channel (2) can be replaced by the central channel (331) in the focusing structure described hereinafter. The central channel (331) is coaxially arranged with the main channel (2) and both ends are connected to the main channel (2). In this sense, the central channel (331) may be considered as a part of the main channel (2) with a different cross-sectional width. In one embodiment, $W_Z=45$

$\mu$m, so the width of the main channel (2) ($2W_Z$) is 90 $\mu$m.

Focusing-separating unit

The number of the focusing-separating unit (3) in a chip may be one, more preferably two or more (for example, 1-20, preferably 1, 2, 3, 4, 5, 6, 7, 8, 9, 10). When there are two or more focusing-separating units 3, different focusing-separating units 3 may be arranged in series and connected to each other through the main channel (2). Different focusing-separating units may have identical or different size parameters, preferably have identical size parameters. The focusing-separating unit (3) comprises at least one collecting port (31), a narrow segment (32) of the main channel, one or more (for example, 1-20, preferably 1, 2, 3, 4 , 5, 6, 7, 8, 9, 10) of series-arranged focusing structures (33) and a separating channel (34). Wherein, sample is inputted through the collecting port (31), especially samples with high flow velocity and high flow discharge. The sample flows into the focusing-separating unit (3) through the main channel (2). After flowing out of one focusing-separating unit (3), the sample may flow into the next focusing-separating unit (3). After flowing out from the last focusing-separating unit (3), a sample with reduced flow velocity and flow discharge is obtained. Preferably, when the chip comprises a capturing unit (4), the sample enters the capturing unit (4) after passing the focusing-separating units.

In the present invention, the cross section of all channels (including the main channel (2), the central channel (331), the branch channel (332) and the separating channel (34), etc.) may be in any suitable shape, such as circular, oval, square, rectangular, or other arbitrary polygons, etc.

Collecting port

The collecting port (31) is used to introduce the sample from the main channel (2) into the narrow segment (32) of the main channel, and preferably forms a funnel-shaped structure, where the cross-sectional width of the widest part of the collecting port is $W_1$, and $W_1$ is not less than the cross-sectional width of the main channel (2) $W_Z$ ($W_Z$ is half of the cross-sectional width of the main channel). In one embodiment, $W_1=2W_Z$; in another embodiment, $W_1>2W_Z$, and in a particular embodiment, $W_1=200$ $\mu$m.

Narrow segment of the main channel

The narrow segment (32) of the main channel is located between the collecting port (31) and the main channel (2), and its cross-sectional width $W_2$ is smaller than the widest part of the collecting port ($W_1$), and also smaller than the width of the main channel ($2W_Z$), thereby locally forming a structure with wide ends and narrow middle.

FIG. 3 shows the diagram of the sample flowing into the main channel (2) through the narrow segment (32) in a particular embodiment. In which, d1 is the distance between the center (or called the center of mass, centroid) of the target particles immediately adjacent to the right side boundary (similarly, also applicable to the left side) of the narrow segment and the right side boundary, and d2 is the distance between the centroid of the target particles closest to the right side boundary of the main channel and the right side boundary of the main channel. If $r_{cell}$ is the radius of the target particles, for target particles with a certain rigidity (such as cells, especially cancer cells, e.g. CTC, etc.), d1 may be considered equal to $r_{cell}$, because the target particles are difficult to be deformed. In actual calculations, in order to ensure that the designed channel prevents all target particles from being separated by mistake, d1 may be slightly smaller than $r_{cell}$. The so-called "slightly smaller" is understood by those skilled in the art, that is, as long as d1 is less than $r_{cell}$, it ensures that the target particles enter the central channel in theory. For example, the value of d1 may be more than 90% of $r_{cell}$, such as 90%, 91%, 92%, 93%, 94%, 95%, 96%, 97%, 98%, 99%, or a value between any two above.

When the liquid flows into the main channel (2) (with normal width) from the narrow segment (32), according to the principle of fluid mechanics, it can be concluded that $d2=2W_Z/W_2*d1$, and the same applies to the cells on the left side boundary. The liquid then further flows into the focusing structure.

Focusing structure

The focusing structure (33) comprises a branch channel (332) and a central channel (331). Preferably, there are two branch channels 332, which are arranged on both sides of the central channel (331) and are symmetrically distributed, and preferably share the same structure and size parameters. Each branch channel (332) intersects the main channel (2) and the central channel (331) at the upper and lower ends (or called the front and back ends). Preferably, the sectional width of the central channel (331) is smaller than the sectional width of the main channel (2), for example, the sectional width of the center channel 331 is 30%-99% of the sectional width of the main channel (2), such as 95%, 90%, 85%, 80%, 75%, 70%, 65%, 60%, 55%, 50%, 45 %, 40%, 35%, 30%, or a value between any two above. After the sample (such as blood) flows through the main channel, a part of the liquid (which contains the target particles) continues flowing into the central channel, while the remaining liquid (which does not contain the target particles) flows into the branch channels on both sides. Then the liquid from the branch channels merges with the liquid flowing out of the central channel and flows into the main channel again. The liquid will enter the next focusing structure or flow away through the separating channel (after the last focusing structure). In addition, in one embodiment, each branch channel (332) may further comprise a branch structure, that is, a form of multiple channels in parallel or a combination of series and parallel.

As shown in FIG. 4A, when the sample flows through the narrow segment (32) of the main channel and enters the first focusing structure (33), a part of the liquid flow in the main channel (2) away from the center will flow into the branch channel (332) in both sides and then merges with the liquid flow out of the central channel (331). Therefore, in order to push the target particles, which are disorderly distributed in the liquid flow, to the center of main channel (2) through the focusing structure (33) (that is, to focus the target particles toward the central part), it is necessary to ensure that the separated liquid does not contain the target particles. All target particles flow through the central channel (331), and the target particles near the boundary of the central channel (331) are pushed by the left/right sidewall of the channel to the center of the channel for a certain distance, and then the separated liquid flows and merges with the liquid flow containing the target particles. In general, after flowing through the focusing structure, the centroid of the cells is pushed close to the center of the flow. In order to achieve the above goals, the following technical solution is applied in the present invention.

Taking the liquid flow in the main channel (2) that will subsequently flow into the branch channel (332) as the observation object. If the distance from the boundary (close to the center of main channel (2)) of the separated liquid flow to the center is r, the liquid flow velocity on the boundary (at distance r from the center of main channel) is v, $v_{max}$ is the velocity at the center of the main channel (2) and also the maximum velocity on the section of the liquid flow in the main channel, and Wz is half the width of the main channel, according to the principles of fluid mechanics, v and $v_{max}$ satisfy the following formula:

$$v = v_{max} \left(1 - \left(\frac{r}{W_z}\right)^2\right) \quad (I)$$

By integrating the velocity distribution and r, the original function is obtained as follows:

$$\int dr = v_{max} r - \frac{1}{3}\frac{v_{max}}{W_z{}^2}r^3 \quad (II)$$

By integrating the entire channel from -$W_Z$ to $W_Z$, the overall flow rate is obtained as shown in the following formula:

$$\int_{-W_Z}^{W_Z} dr = \frac{4}{3}v_{max}W_Z \quad (III)$$

Next, by integrating from -r to r, the flow rate of the liquid remaining in the main channel after flowing through the main channel can be calculated, as shown in the following formula:

$$\int_{-r}^{r} dr = v_{max}\left(2r - \frac{2}{3}\frac{r^3}{W_z{}^2}\right) \quad (IV)$$

If the shunting ratio is k, that is, the ratio of the flow rate entering the branch channel (332) to the overall flow rate, it is known that the ratio of the liquid flow remaining in the main channel to the overall flow is 1-k, as shown in the following formula:

$$1\text{-}k = v_{max}\left(2r - \frac{2}{3}\frac{r^3}{W_z{}^2}\right) \Big/ \frac{4}{3}v_{max}W_Z = \frac{3}{2}\frac{r}{W_z} - \frac{1}{2}\frac{r^3}{W_z{}^3} \quad (V)$$

From the above, it can be known that the distance between the centroid of the cells closest to the right side (or left side) wall of the main channel that flows through the narrow segment of the main channel and the right side (or left side) wall of the main channel is d2, where $d2=2W_Z/W_2*d1$ (FIG. 3), and $W_2$ represents the width of the narrow segment of the main channel. For the liquid flowing through the narrow segment of the main channel and about to enter the first

$$r = W_Z - d2 = \frac{W_2 W_Z - 2d1 W_Z}{W_2}$$

focusing structure, at this time, , which is then brought into the formula (V) to obtain

$$1-k = \frac{3}{2}\frac{W_2-2d1}{W_2} - \frac{1}{2}\left(\frac{W_2-2d1}{W_2}\right)^3 \quad (VI).$$

R1 is the flow resistance of the central channel (331) of the first focusing structure, and R2 is the flow resistance of the branch channel (332) of the first focusing structure. The flow resistance of the channel is related to the dimensional characteristics of the channel. In the present invention, when the proportional relationship of the flow resistance is calculated, the calculation formula of flow resistance is as follows: the flow resistance $R_{flow\ resistance}$ is expressed as: $R_{flow\ resistance} = 8\eta L(H+W)^2/(HW)^3$, where $\eta$ represents the viscosity coefficient of the liquid, and L, W and H respectively represent the length, width, and height of the channel, such as the central channel and the branch channel. According to the inverse proportion of the flow resistance to the flow rate distribution, the flow rate distribution ratio can be calculated based on the flow resistance

$$1-k = \frac{R2}{R2+2R1} \quad (VII).$$

In order to ensure that the target particles continue remaining in the main channel, and the liquid flow containing no target particles is separated as much as possible, the following formula needs to be met:

$$\frac{3}{2}\frac{W_2-2d1}{W_2} - \frac{1}{2}\left(\frac{W_2-2d1}{W_2}\right)^3 \leq \frac{R2}{R2+2R1} \quad (VIII),$$

wherein the left side of the inequality represents the ratio of the liquid flow corresponding to the area where the centroid of cells is located to the total liquid flow, and the right side represents the ratio of the liquid flow that continues to remain in the main channel to the total liquid flow calculated according to the flow resistance of the first focusing structure. The right side needs to be slightly greater than or equal to the left side to ensure that the target particles will not be out of the main channel.

In order to ensure that the target particles can be further concentrated when flowing through all the focusing structures, it is necessary that the flow resistances of the front and back focusing structures meet a certain relationship. The flow resistance of the central channel of the $n^{th}$ (n is greater than or equal to 1 and less than or equal to the total number of focusing structures minus 1) focusing structure is set as $R1^n$, the flow resistance of the branch channel is set as $R2^n$, the flow resistance of the central channel of the $(n+1)^{th}$ focusing structure is set as $R1^{n+1}$, and the flow resistance of the branch channel is set as $R2^{n+1}$. After flowing through the $n^{th}$ focusing structure, the distance from the center of the target particles to the boundary is: $(W_Z-r+p)$, where p represents the distance that the target particles originally located at the boundary move to the center after flowing through the $n^{th}$ focusing structure. In order to ensure that when flowing through the $(n+1)^{th}$ focusing structure, all target particles are further concentrated and no target particles enter the branch channel, the following formula needs to be met:

First finding out the ratio of the flow rate of the area between the centroid of particles closest to the left side and the centroid of particles closest to the right side in the central channel to the flow rate of the central channel (FIG. 4A). Based on similar principles, according to the formula V,

$$\text{this ratio} = \frac{3}{2}\frac{r}{W_Z} - \frac{1}{2}\frac{r^3}{W_Z{}^3},$$

but to replace r with $Ws^n/2-d1$ and $W_Z$ with $Ws^n/2$, where $Ws^n$ represents the width of the central channel of the $n^{th}$ focusing structure (n is greater than or equal to 1 and less than or equal to the total number of focusing structures minus 1). The specific expression of the above ratio is further obtained as follows:

$$\frac{3}{2}\frac{W_s{}^n-2\mathrm{d}1}{W_s{}^n} - \frac{1}{2}\frac{(W_s{}^n-2\mathrm{d}1)^3}{(W_s{}^n)^3}.$$

Based on the ratio of the flow rate of the center channel to the overall flow rate of the main channel 1-k, similarly, according to formula (VII),

$$1\text{-}k = \frac{R2^n}{R2^n+2R1^n}$$

can be obtained, and the ratio of the flow rate of the area between the centroid of particles closest to the left side and the centroid of particles closest to the right side in the central channel to the flow rate of the central channel can be obtained as

$$\frac{R2^n}{R2^n+2R1^n}\left(\frac{3}{2}\frac{W_s{}^n-2\mathrm{d}1}{W_s{}^n} - \frac{1}{2}\frac{(W_s{}^n-2\mathrm{d}1)^3}{(W_s{}^n)^3}\right).$$

Further, according to formula (VII), it can be calculated that when the liquid flow is about to enter the $(n+1)^{th}$ focusing structure, the ration of the flow rate continuing to remain in the main channel to the overall flow rate is 1-k =

$$\frac{R2^{n+1}}{R2^{n+1}+2R1^{n+1}}.$$

. In order to ensure that the target particles continue remaining in the main channel, and the liquid flow containing no target particles is separated as much as possible, the followings need to be met:

$$\frac{R2^n}{R2^n+2R1^n}\left(\frac{3}{2}\frac{W_s{}^n-2\mathrm{d}1}{W_s{}^n} - \frac{1}{2}\frac{(W_s{}^n-2\mathrm{d}1)^3}{(W_s{}^n)^3}\right) \leq \frac{R2^{n+1}}{R2^{n+1}+2R1^{n+1}} \quad (IX)$$

.

The left side of the inequality represents the ratio of the liquid flow corresponding to the area where the centroid of cells is located to the total liquid flow, and the right side represents the ratio of the liquid flow that continues remaining in the main channel to the total liquid flow calculated according to the flow resistance of the $(n+1)^{th}$ focusing structure. The right side needs to be slightly greater than or equal to the left side to ensure that the target particles will not be separated.

Combining formula (VIII) and formula (IX), for target particles (such as tumor cells) of different sizes, the specific size and proportional relationship of the focusing structure that can achieve the goal of continuous focusing of target particles may be determined, but not limited to specific size and proportional relationships.

Separating channel

The function of the separating channel (34) is that after the concentration of one or more focusing structures (33), the target particles have been pushed close to the center of the liquid flow. By establishing the separating channel (34) connected to the main channel (2), a part of the liquid flow that does not contain target particles is discharged, thereby reducing the flow velocity and flow discharge of the liquid flow, then enters the next focusing-separating unit (3), and will enter the capturing unit (4) through the main channel (2) after the sample flows through the last focusing-separating unit (3), if the target particles need to be captured.

The design principle of the separating channel is: after the concentration of the focusing structure (33) and before flowing into the separating channel (34), the target particles in the main channel have concentrated in the range close to the center of the liquid flow (FIG. 5A). Therefore, in order to reduce the flow velocity and flow discharge, it is only necessary to discharge the part of the liquid flow near the boundary, which does not contain the target particles.

There are preferably two separating channels (34), preferably symmetrically arranged on both sides of the main channel (2). Each separating channel (34) intersects with the main channel upstream and downstream (or at the front and back ends), and the liquid entering the separating channel (34) goes back to the main channel (2) at the downstream intersection and is discharged from the outlet (5). In one embodiment, each single separating channel (34) may further comprise a branch structure, that is, a form of multiple channels in parallel or a combination of series and parallel.

In one embodiment, as shown in FIG. 5B, when the liquid flows through one or more focusing structures and then flows into the main channel (2), the distance between the center point of the liquid flow and the channel boundary (such as the right boundary) is set as Wz. Meanwhile, the target particles are all pushed near the center of the liquid flow due to the concentration effect, the distance between the boundary of the liquid flow containing the target particle and the center point is set as r. Therefore, the width of the liquid flow that needs to be separated should be less than or equal to Wz-r.

In an exemplary embodiment, the total width of the main channel (2) is 90 $\mu$m, that is, Wz=45 $\mu$m. Each focusing-separating unit (3) comprises five focusing structures (33). After being concentrated, the target particles are concentrated in an interval of [-16 $\mu$m, 16 $\mu$m], that is, r=16 $\mu$m (taking the center point of the channel as the origin of the coordinates), so $W_Z$-r =29 $\mu$m. Therefore, the optimal design is to let part of the liquid (with a width of 29 $\mu$m) flowing away through both sides, so as to ensure that as many non-target particles (such as white blood cells and red blood cells) can be removed as much as possible, while retaining target particles (such as CTCs).

In order to ensure that the liquid flow with a width of $W_Z$-r splits out, it is necessary to determine how much proportion of the liquid flow needs to enter the separating channel, so the following calculations are required. The following calculation process is similar to the calculation process that calculates the separated flow rate of the branch channel in the focusing structure. The details are as follows.

Taking the liquid flow entering in the main channel (2) that is away from the center of the channel and will subsequently flow into the separating channel (34) as the observation object. If the distance between the boundary (close to the center of the main channel) of the liquid flow and the center is r (the same as the distance between the boundary of the part of the liquid flow containing the target particle and the center point r), the liquid flow velocity on the boundary is v, $v_{max}$ is the flow velocity of the liquid flow at the center of the main channel and also the maximum velocity on the section of the liquid flow, and $W_Z$ is half the width of the main channel, according to the principles of fluid mechanics, v and $v_{max}$ satisfy the following formula:

$$v = v_{max} \left(1 - \left(\frac{r}{W_Z}\right)^2\right) \quad \text{(I)}.$$

By integrating the velocity distribution and r, the original function is obtained as follows:

$$\int dr = v_{max}\, r - \frac{1}{3}\frac{v_{max}}{W_Z{}^2}\, r^3 \quad \text{(II)}.$$

By integrating the entire channel from -$W_Z$ to $W_Z$, the overall flow rate is obtained as shown in the following formula:

$$\int_{-W_Z}^{W_Z} dr = \frac{4}{3} v_{max} W_Z \quad \text{(III)}.$$

Next, by integrating from -$W_Z$ to -r and from r to $W_Z$, the liquid flow that is separated into the separating channels on both sides after flowing into the main channel can be calculated, as shown in the following formula:

$$2\int_{-W_Z}^{-r} dr = v_{max}\left(\frac{4}{3}W_Z - 2r + \frac{2}{3}\frac{r^3}{W_Z{}^2}\right) \quad \text{(X)}.$$

If the shunting ratio is k, that is, the ratio of the flow rate entering the separating channel to the total liquid flow rate, the calculation of k is shown in the following formula:

$$k = v_{max}(\frac{4}{3}W_Z - 2r + \frac{2}{3}\frac{r^3}{W_Z^2}) \ / \ \frac{4}{3}v_{max}W_Z \ = 1 - \frac{3}{2}\frac{r}{W_Z} + \frac{1}{2}\frac{r^3}{W_Z^3} \quad (XI).$$

Also in the foregoing exemplary embodiment, where $W_Z=45$ μm and $r = 16$ μm. After putting $W_Z$ and $r$ into formula (XI), it can be calculated that the ratio of k is about 0.44.

Wherein, the liquid distribution follows the formula:

$$\frac{\text{Separated liquid flow}}{\text{Liquid flow remaining in the main channel}} = \frac{\text{Overall flow resistance of the internal area of the separating channel}}{\text{Overall flow resistance of the separating channels on both sides (parallel)}}$$

For each time of shunting, set R1 as the flow resistance of the internal area of the separating channel (34) (see below for the explanation), and R2 as the flow resistance of the separating channels on both sides, and the flow resistance of the separating channel is calculated as follows: $R_{flow\ resistance} = 8\eta L(H+W)^2/(HW)^3$, where $\eta$ represents the viscosity coefficient of the liquid, and L, H and W respectively represent the length, height, and width of the channel. Based on the above method, the shunting ratio $k = \dfrac{R1}{R1+1/2R2}$.

In various embodiments of the present invention, the microfluidic chip comprises multiple groups of focusing-separating units, which may be arranged in series up and down. For each specific shunting part, the flow resistance of the internal area of the separating channel (34) refers to the overall flow resistance of all channels or other structures in the area between the two intersections of the separating channel (34) and the main channel (2). For brevity, it is also referred to as "flow resistance between intersections" or "flow resistance of internal intersection", and is sometimes abbreviated as "overall internal flow resistance" or "internal flow resistance". The calculation process is given below, starting from the last group, that is, the focusing-separating unit connected to the capturing unit.

Set the flow resistance of internal intersection of the $m^{th}$ group (m is a natural number greater than or equal to 1 and less than or equal to the total number of focusing-separating units) of focusing-separating units as $R1^m$, for the last focusing-separating unit (that is, m is equal to the number of focusing-separating unit), the internal overall flow resistance includes the flow resistance of the connecting channel (41) connecting the capture area and the main channel plus the flow resistance of the small blocks (42) of the capturing unit. The flow resistance of the separating channels on both sides of the shunting part is $R2^m$. Since the ratio k that needs to be satisfied is known (each focusing-separating unit satisfies the same k), according to the formula k

$$= \frac{R1^m}{R1^m+1/2R2^m},$$

the relationship between $R1^m$ and $R2^m$ can be calculated, that is,

$$\frac{k}{1-k} = \frac{2R1^m}{R2^m},$$

so that

$$R2^m = \frac{2(1-k)}{k}R1^m,$$

where for simplicity, let

$$x = \frac{2(1-k)}{k}.$$

Set the overall flow resistance of the separating channels on both sides of the m-1 group of shunting part as $R1^{m-1}$, and the flow resistance of the separating channels on both sides as $R2^{m-1}$. According to the formula

$$k = \frac{R1^{m-1}}{R1^{m-1}+1/2R2^{m-1}},$$

the relationship between $R1^{m-1}$ and $R2^{m-1}$ can be calculated. In addition, because $R1^{m-1}$ is actually generated by the parallel connection of $R1^m$ and two $R2^m$ in the shunting part of the behind group it, the flow resistance of the two groups of $R2^m$ in parallel is $1/2*R2^m$, and it is further calculated that $R1^{m-1}=R1^m*1/2*R2^m/(R1^m+1/2R2^m)$, where $R2^m=x*R1^m$, according to the above formula, $R1^{m-1}=[x/(x+2)]*R1^m$ (XII).

By analogy, the specific parameters of R1 and R2 of the shunting part in each group of focusing-separating units can be calculated, so as to determine the requirements that should be met, for example, the length, width and height of the separating channel and the central channel.

In one embodiment, k=0.5 (i.e., half of the flow rate of the liquid flow is discharged).

In a particular embodiment, the chip comprises a total of 7 groups of focusing-separating units 3, and each focusing-separating unit (3) comprises a group of separating channels (34) (one separating channel (34) on the left side and one separating channel (34) on the right side of the main channel (2), the two arranged in parallel), a total of 7 groups of separating channels (34).

For the separating channel (34) of the last focusing-separating unit, the liquid flow will be distributed according to the flow resistance of the internal separating channel and the parallel flow resistance of the separating channel. The flow resistance of the internal separating channel (34) is the flow resistance of the capturing unit, which is set as $R1^7$, and the unilateral flow resistance of the separating channels (34) on both sides is set as $R2^7$. In order to ensure that half of the flow can be separated, the total flow resistance of the separating channels on both sides (parallel) is required to be equal to the flow resistance of the capturing unit $R1^7$. The calculation is as follows:

The flow resistance of the separating channels (parallel) on both sides is: $(R2^7* R2^7)/(R2^7+R2^7) = 1/2*R2^7 = R1^7$, therefore:

$R2^7 = 2R1^7$, that is, x=2 in formula XII.

It can be easily calculated that the total flow resistance of the internal area of the second, third, fourth, fifth, sixth, seventh group of the shunting part from the bottom is about $1/2* R1^7$, $1/4* R1^7$, $1/8* R1^7$, $1/16* R1^7$, $1/32* R1^7$ and $1/64* R1^7$;

The flow resistance of the separating channel of the second, third, fourth, fifth, sixth, and seventh group of the shunting part from the bottom is about $R1^7$, $1/2* R1^7$, $1/4* R1^7$, $1/8* R1^7$, $1/16 * R1^7$ and $1/32* R1^7$.

Based on the above calculations, the flow resistance relationship of each separating channel and the specific size ratio design can be easily obtained.

Capturing unit

There are a variety of capture structures for capturing target particles such as tumor cells in the prior art. In theory, all existing capture structures may be used as the capturing unit (4) of the present invention. Preferably, the chip of the present invention adopts a multi-layer of gap design between the entering of the liquid flow containing the target particles and the flowing out of the capturing unit (4), which is connected to the outlet (5) at the end, so that the liquid flow after capturing is discharged from the outlet (5). The gaps through which the liquid flows gradually become smaller, so as to specifically capture target particles of a specific size. Preferably, the size of the target particles is larger than the non-target particles in the liquid flow, and more preferably, the target particles are tumor cells, such as circulating tumor cells.

In one embodiment, the capturing unit (4) is an array structure with multiple layers (for example, 3-10 layers, specifically 1, 2, 3, 4, 5, 6, 7, 8, 9, 10 layers), preferably, each array is formed by arranging small blocks of any shape (such as cube, rectangular parallelopiped, triangular prism, cylinder, etc., or with a section of square, rectangle, triangle, circle, etc.), and there is a gap between every two small blocks, wherein the gap is defined as the closest distance between the two adjacent small blocks; also preferably, the gap between each layer of the array is identical; further preferably, the gap between different layers of the array is gradually decreased from top to bottom, for example, when the number of layers of the array is 4, the gap from top to bottom is 14 $\mu$m, 12 $\mu$m, 10 $\mu$m and 8 $\mu$m.

In one embodiment, the capturing unit (4) is designed as a structure comprising four layers of triangular arrays, the sizes of the gaps from top to bottom are 14 $\mu$m, 12 $\mu$m, 10 $\mu$m, and 8 $\mu$m, and each layer includes 3, 3, 3, 5 rows of evenly arranged triangles. The triangles are equilateral triangles with a side length of 80 $\mu$m.

In a particular embodiment, the capturing unit (4) is used to capture tumor cells, especially CTCs, because CTCs are large in size and not easily deformed, the cells will be stuck in the gaps, while other blood cells such as white blood cells, which have a relatively small size and strong deformability, will pass through the gaps freely.

Example 1 Chip design

As shown in FIG. 1, the microfluidic chip prepared in this example comprises both a focusing-separating unit (3) and a capturing unit (4), wherein the number of the focusing-separating units 3 is seven, and the units are arranged in series. Each focusing-separating unit (3) comprises a collecting port (31), a narrow segment (32) of the main channel, five focusing structures (33), and two separating channels (34) arranged on both sides.

In which, the sectional diameter of the widest part of the collecting port (31) is $W_1$ = 200 $\mu$m, and the whole presents a funnel-shaped structure. The sectional diameter of the narrow segment (32) of the main channel is $W_2$ = 30 $\mu$m. The sectional diameter of the main channel (2) is 90 $\mu$m.

For each focusing-separating unit (3), the branch channel (332) of the five focusing structures (33) have widths of 30, 40, 40, 40, and 40 $\mu$m respectively, and lengths of 1180, 900, 600, 440, and 440 $\mu$m (length of one side) respectively. The width of the center channel 331 Ws is 30 $\mu$m, and the width of the main channel (2) 2$W_Z$ is 90 $\mu$m.

The seven focusing-separating units 3 have the same number and size of focusing structure (33), while the separating channels (34) have widths of 220, 200, 180, 160, 140, 110, and 85 $\mu$m from top to bottom, and lengths of 75, 131, 70455, 65809, 61313, 56917, 52691 and 48570 $\mu$m (length of one side) respectively.

Preparation process

(1) For specific target particles, based on theoretical calculations, the parameters of the focusing-separating unit and the capturing unit were designed, and the chip layout was drawn using L-edit;

(2) A mask was made using chromium plate, and the mold was made by using silicon wafer or chromium plate as substrate, su-8 photoresist to coat, pre-baking, exposure, post-baking, and developing;

(3) PDMS A gel: B gel = 8:1 were employed and mixed well and poured into the mold, and heating and curing were carried out to finish the chip. After the chip was punched at desired positions, it was bonded with the slide after air plasma treatment.

Example 2 Capture of tumor cells in the blood by the chip

The chip was pre-filled with PBS to exhaust the air in the channels, and then incubated with 1% BSA for 0.5 h to prevent cell adhesion.

HeLa cells were digested, stained with CellTracker, and diluted to a concentration of 10,000 cells/mL. Rabbit blood was added with an equal volume of heparin (10 mg/mL) to 1:1 dilution to prevent coagulation.

1 ml of diluted blood was added with 100 $\mu$L of stained HeLa cells with a concentration of 10,000/mL, and the sample was injected into the chip with a syringe pump while controlling the flow rate, and the outlet was connected to a 24-well plate.

The focusing and separating areas at all levels of the chip were observed under a fluorescence microscope. HeLa cells and other cells being focused and separated in the flow were summarized and plotted (FIG. 4B and 4C). The results confirmed that the tumor cells gradually moved to the center after flowing through focusing structure. It was also observed that red blood cells and white blood cells were separated away by the separating channel, while tumor cells were not (FIG. 5C, fluorescence signal indicated tumor cells, and cells without fluorescence were red blood cells and white blood cells).

Subsequently, a variety of different human tumor cells (HeLa, NCI-H226, MCF-7, and MB-MDA-231) were used for experiments. These tumor cells have similar size, width an average diameter of about 12-16 $\mu$m. The chip of Example 1 was designed based on such parameters, and theoretically can be used for the separation of all these cells. In addition, the flow rate gradient from low to high and different cell density were set for experiments to verify the reliability of the chip.

The calculation formula of the capture efficiency is: capture efficiency (%) = cells captured in the chip/ (cells captured in the chip + cells flowing out of the chip) * 100%. Because the tumor cells were labeled with fluorescence, the number of captured tumor cells was the number of the cells with fluorescence, which was counted from the image of the entire capturing area under a fluorescence microscope. The liquid flowing out of the chip was collected in the 24-well plate. ACK lysis buffer was added to break the red blood cells to make the field of vision clearer. Then images were taken and counting was performed under a fluorescent microscope to obtain the number of tumor cells flowing out.

The results are shown in FIG. 6A, at different flow rates, high capture efficiency on HeLa cells can be maintained. Especially when the flow rate reached 40 mL/h, the capture efficiency was still more than 90%. Even when the flow rate reached 60 mL/h, the capture efficiency still exceeded 85%. FIG. 6B shows the capture efficiency of human tumor cells of different sizes at a flow rate of 40 mL/h. It can be seen that the chip exhibited similar capture efficiencies for different tumor cell lines, all reaching more than 90%. FIG. 6C shows that when the density of tumor cells was different, especially when the cell density was very low, the chip still maintained a stable and high capture efficiency.

Next, the distribution of tumor cells in the capture area at different flow rates was also measured. The results are shown in FIG. 7. As the flow rate increased, the tumor cells gradually moved backwards. When the flow rate increased to 60 mL/h, there were still no tumor cells leaking from the gaps in the capture area, indicating that the chip of the present invention can effectively capture tumor cells.

Example 3 Determination of the residual rate of white blood cells

Leukocyte residue is a phenomenon that often occurs in tumor cell capture chips. If the leukocyte retention rate is too high, it will affect the purity of the separated tumor cells, and then affect the subsequent analysis and detection results. In order to test the residual status of white blood cells in the chip of Example 1, the number of residual white blood cells in multiple different experiments was counted and analyzed (FIG. 8). The results show that the residual rate of white blood cells in the chip of Example 1 was 8.1$\pm$6.6 per 100,000, that is, the average residual white blood cell ratio was 0.008%, which is much lower than other physical separation methods.

Example 4 Clinical sample detection

It is known that there are rare circulating tumor cells (CTCs) in the blood of cancer patients, and early detection of cancer and adjuvant treatment can be achieved through effective capture of blood CTCs. In order to test the efficiency of capturing tumor cells in the blood of cancer patients by the chip of the present invention, in cooperation with the hospital, 2 mL of blood samples of 3 healthy volunteers and 6 diagnosed cancer patients were collected respectively and numbered randomly. The chip of Example 1 was used to capture circulating tumor cells. After the capture, the *in situ* CD45/EpCAM/Hoechst staining was performed on the chip. According to the CellSearch standard, the cells showing Hoechst$^+$CD45$^-$EpCAM$^+$ were CTCs. The result is shown in FIG. 9. Two CTCs per milliliter of blood were detected in all healthy blood samples. The reason why CTCs were also detected in the blood of healthy people is that, EpCAM stains E-type cells, and in addition to CTCs, skin cells and very few blood cells in the blood are also E-type cells, resulting in contamination in the result. However, in the blood samples of all cancer patients, significantly more CTCs were captured, especially in the two patients with stage IV breast cancer, 117 and 47 CTCs were detected in each milliliter of blood. In patients with stage III and stage IV lung cancer, 30-40 CTCs were detected in each milliliter of blood, while in patients with earlier lung cancer (IIB), 17 CTCs were detected per milliliter of blood. The above data shows that the chip of the present invention can effectively capture CTCs in clinical samples with high sensitivity, giving immeasurable clinical application value.

**Claims**

1. A microfluidic chip comprising an inlet (1), a main channel (2), one or more (for example, 1-20, preferably 1, 2, 3, 4, 5, 6, 7, 8, 9 or 10) of focusing-separating units and an outlet (5), wherein the inlet (1), the focusing-separating unit (3) and the outlet (5) are connected by the main channel (2);
   when a liquid sample enters the main channel (2) from the inlet (1) and flows through the focusing-separating unit (3), the focusing-separating unit (3) pushes target particles in the sample to the center of the liquid flow, and simultaneously discharges or partially discharges the liquid flow that does not contain the target particles from the outlet (5), thereby reducing the flow velocity and/or flow discharge of the sample without losing the target particles.

2. The microfluidic chip according to claim 1, wherein the focusing-separating unit (3) comprises a collecting port (31), a narrow segment (32) of the main channel, one, two or more (for example, 1-20, such as 1, 2, 3, 4, 5, 6, 7, 8, 9, 10) of series-arranged focusing structure (33), and a separating channel (34).

3. The microfluidic chip according to claim 2, wherein the focusing structure (33) comprises a central channel (331) and a branch channel (332), wherein

the central channel (331) is connected to the main channel (2) at both ends and arranged coaxially; preferably, the width of the central channel (331) is smaller than the width of the main channel (2), and preferably, the width of the central channel (331) is 30%-99% of the width of the main channel (2), such as 95%, 90%, 85%, 80%, 75%, 70%, 65%, 60%, 55%, 50%, 45%, 40%, 35%, 30%, or a value between any two above; and
the branch channel (332) intersects the main channel (2) and the central channel (331) at both ends; further preferably, there are two branch channels (332); further preferably, the two branch channels (332) are arranged at both sides of the central channel (331), more preferably, the two branch channels (332) are arranged symmetrically at both sides of the central channel (331), and share the same size parameters.

4. The microfluidic chip according to claim 3, wherein the flow resistance of the central channel (331) has a proportional relationship with the flow resistance of the branch channel (332), when the sample enters the focusing structure (33) from the main channel (2), the liquid flow containing the target particles enters the central channel (331), the liquid flow containing no target particles flows into the branch channel (332) and merges with the liquid flow from the central channel (331), and then flows into the main channel (2) again.

5. The microfluidic chip according to claim 4, wherein the proportional relationship of the flow resistance is based on the ratio of $L(H+W)^2/(HW)^3$ of the channels, and L, W and H respectively represent the length, width and height of the channels.

6. The microfluidic chip according to any one of claims 3 to 5, wherein when Wz represents half the width of the main channel (2), $W_2$ represents the width of the narrow segment (32) of the main channel, $r_{cell}$ represents the average radius of the target particles in the sample, d1 represents the distance between the centroidcentroid of the target particles closest to one side boundary of the narrow segment (32) of the main channel in the liquid flow and the side boundary, R1 is the flow resistance of the central channel (331) of the first focusing structure (33) in each focusing-separating unit (3), and R2 is the flow resistance of a single branch channel (332) in the first focusing structure (33), the following formula is met:

$$\frac{3}{2}\frac{W_2-2d1}{W_2} - \frac{1}{2}\left(\frac{W_2-2d1}{W_2}\right)^3 \leq \frac{R2}{R2+2R1}$$ (VIII), wherein d1 is equal to $r_{cell}$, or slightly smaller than $r_{cell}$.

7. The microfluidic chip according to any one of claims 3 to 6, wherein if the flow resistance of the central channel (331) of the $n^{th}$ (n is a natural number greater than or equal to 1 and less than the total number of the focusing structures) focusing structure (33) is $R1^n$, the width is $W_s^n$, the flow resistance of a single branch channel (332) is $R2^n$, the flow resistance of the central channel (331) of the $(n+1)^{th}$ focusing structure (33) is $R1^{n+1}$, and the flow resistance of each branch channel (332) is $R2^{n+1}$, the following formula is met:

$$\frac{R2^n}{R2^n+2R1^n}\left(\frac{3}{2}\frac{W_s^n-2d1}{W_s^n} - \frac{1}{2}\frac{(W_s^n-2d1)^3}{(W_s^n)^3}\right) \leq \frac{R2^{n+1}}{R2^{n+1}+2R1^{n+1}}$$ (IX), wherein d1 is equal to $r_{cell}$, or slightly smaller than $r_{cell}$.

8. The microfluidic chip according to any one of claims 2 to 7, wherein the separating channel (34) intersects with the main channel (2) at both ends, and upstream intersection is close to the last focusing structure in the focusing-separating unit, and downstream intersection is close to the outlet (5) of the microfluidic chip; preferably, there are two separating channels (34) arranged at both sides of the main channel (2); more preferably, the two separating channels (34) are arranged symmetrically at both sides of the main channel (2) and share the same size parameter; also preferably, at the intersection of the separating channel (34) and the main channel (2), the width of the main channel becomes larger, for example, the width is 1.5-5 times of the original width, such as 1.5 times, 2 times, 2.5 times, 3 times, 3.5 times, 4 times, 4.5 times or 5 times, or a multiple value between any two above; further preferably, the sidewall of the channel at the intersection is rounding.

9. The microfluidic chip according to claim 8, wherein the flow resistance of the separating channel (34) has a proportional relationship with the overall flow resistance of the area between the two intersections of the separating channel (34) and the main channel (2), so that the target particles continue flowing into the main channel (2), and the liquid flow without the target particles flows into the separating channel (34).

10. The microfluidic chip according to claim 8 or 9, wherein the flow resistance of the separating channel (34) of the m^th (m is a natural number greater than or equal to 1, and less than or equal to the total number of the focusing-separating units, such as m = 1, 2, 3, 4, 5, 6 , 7, 8, 9 or 10) focusing-separating unit (3) is determined as follows: the overall flow resistance of the area between the two intersections of the separating channel (34) and the main channel (2) of the m^th focusing-separating unit (34) (or the overall flow resistance of the internal area of the separating channel)

$$R2^m = \frac{2(1-k)}{k} R1^m$$

is R1^m, and the flow resistance of each separating channel (34) is R2^m, , wherein k is the ratio of the liquid flow in the separating channel (34) to the overall liquid flow.

11. The microfluidic chip according to any one of claims 1 to 10, wherein the microfluidic chip further comprises a capturing unit (4) for capturing target particles; preferably, the capturing unit (4) is located downstream of the focusing-separating unit (3), and both ends are connected to the main channel (2); also preferably, the capturing unit is connected to the main channel (2) through a branch channel (41).

12. The microfluidic chip according to claim 11, wherein the capturing unit (4) comprises one layer or more than one layer (for example, 1, 2, 3, 4, 5, 6, 7, 8, 9 or 10 layers) of array, preferably, the array is formed by arranging small blocks (42) of any shape (such as cube, rectangular parallelopiped, triangular prism, cylinder, etc., or with a cross section of square, rectangle, triangle, circle, etc.), and there is a gap d between adjacent small blocks (42), wherein the gap d is defined as the closest distance between the surfaces of two adjacent small blocks (42); also preferably, the gap d between the small blocks (42) in each layer of the array is identical; further preferably, the gap d between the small blocks (42) in different layers of the array is gradually decreased from top to bottom, for example, when the number of layers of the array is 4, the gap d from top to bottom is 14 μm, 12 μm, 10 μm and 8 μm.

13. The microfluidic chip according to any one of claims 1 to 12, wherein the inlet (1) further comprises a filtering structure (11).

14. A device for enriching and/or capturing target particles in a sample, comprising the microfluidic chip according to any one of claims 1 to 13.

15. A method for reducing the flow discharge and/or flow velocity of a sample, comprising using the microfluidic chip according to any one of claims 1 to 13 or the device according to claim 14.

16. A method for enriching and/or capturing target particles, comprising:

(1) providing a liquid sample containing target particles; and
(2) injecting the liquid sample into the microfluidic chip according to any one of claims 1 to 13 or the device according to claim 14.

17. A method for removing target particles in a liquid sample, comprising:

(1) providing a liquid sample containing target particles;
(2) injecting the liquid sample into the microfluidic chip according to any one of claims 1 to 13 or the device according to claim 14; and
(3) collecting the liquid sample flowing out from the outlet (5) of the microfluidic chip; optionally, repeating steps (2) and (3), and the repeating is performed 1, 2, or more times (for example, 3, 4, 5, 6, 7, 8, 9 or 10 times).

18. The method according to any one of claims 15 to 17, wherein the liquid sample is injected at a rate of 5-200 mL/h, 10-150 mL/h, 20-100 mL/h, 30-80 mL/h, 40-60 mL/h, specifically, the rate is 10, 20, 30, 40, 50, 60, 70, 80, 90, 100, 110, 120, 130, 140, 150, 160, 170, 180, 190 or 200mL/h, or a value between any two above.

19. The chip according to any one of claims 1 to 13, the device according to claim 14, and the method according to any one of claims 15 to 18, wherein the sample is whole blood, plasma, serum, perfusion fluid, urine, tissue fluid, cerebrospinal fluid, cell culture fluid or cell mixture, and preferably, the sample is whole blood or perfusion fluid.

20. The chip according to any one of claims 1 to 13, the device according to claim 14, and the method according to any one of claims 15 to 18, wherein the target particles are tumor cells, preferably circulating tumor cells (CTC).

A

B

FIG. 1

**FIG. 2**

W₁ = 200 μm
W₂ = 30 μm
d1
d2
W₃ = 90 μm = 2W_z

**FIG. 3**

A

B

Position of cell centroid
is 14.26µm

Position of cell centroid
is 6.84µm

FIG. 4

FIG. 4 (continued)

**A**

**B**

**FIG. 5**

C

**FIG. 5 (continued)**

**FIG. 6**

**FIG. 7**

**FIG. 8**

Groups 1-3: Healthy volunteer
Groups 4-5: Breast cancer patient
Groups 6-9: Lung cancer patient

**FIG. 9**

## INTERNATIONAL SEARCH REPORT

| International application No. |
|---|
| **PCT/CN2020/102564** |

| A. | CLASSIFICATION OF SUBJECT MATTER |
|---|---|

C12M 1/12(2006.01)i; C12M 1/00(2006.01)i; C12N 5/09(2010.01)i

According to International Patent Classification (IPC) or to both national classification and IPC

| B. | FIELDS SEARCHED |
|---|---|

Minimum documentation searched (classification system followed by classification symbols)

C12M;C12N

Documentation searched other than minimum documentation to the extent that such documents are included in the fields searched

Electronic data base consulted during the international search (name of data base and, where practicable, search terms used)

VEN, CNABS, PubMed, CNKI, ISI Web of Knowledge, 细胞, 循环肿瘤细胞, 流阻, 流动阻力, 微流控芯片, 微通道, 捕获, 分选, 分离, 支流, 分流, 支路, 微道, cell, CTC, flow resistance, microfluidic, chip, cell sorting, captur+ , distributary, bypass , microchannel, applicant, inventor

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 106190774 A (SHENZHEN LABVIV TECHNOLOGY CO., LTD.) 07 December 2016 (2016-12-07) <br> specific embodiments | 1, 11-20 |
| A | CN 107402295 A (SUREXAM BIO-TECH CO., LTD.) 28 November 2017 (2017-11-28) <br> entire document | 1-20 |
| A | CN 204939452 U (SHENZHEN RUISI LIFE TECHNOLOGY CO., LTD.) 06 January 2016 (2016-01-06) <br> entire document | 1-20 |
| A | CN 107723207 A (SHENZHEN RUIGE BIOTECHNOLOGY CO., LTD.) 23 February 2018 (2018-02-23) <br> entire document | 1-20 |
| A | CN 103834558 A (SHENZHEN INSTITUTES OF ADVANCED TECHNOLOGY, CHINESE ACADEMY OF SCIENCES) 04 June 2014 (2014-06-04) <br> entire document | 1-20 |

☑ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 September 2020** | **21 October 2020** |

| Name and mailing address of the ISA/CN | Authorized officer |
|---|---|
| **China National Intellectual Property Administration (ISA/ CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/102564** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 109852530 A (SHANGHAI INSTITUTE OF MICROSYSTEM AND INFORMATION TECHNOLOGY, CHINESE ACADEMY OF SCIENCES) 07 June 2019 (2019-06-07)<br>entire document | 1-20 |
| A | CN 208604119 U (BEIJING FRIENDSHIP HOSPITAL, CAPITAL MEDICAL UNIVERSITY) 15 March 2019 (2019-03-15)<br>entire document | 1-20 |
| A | CN 109852544 A (OUYANG, Dongfang) 07 June 2019 (2019-06-07)<br>entire document | 1-20 |
| A | WO 2014065861 A1 (UNIV. PENNSYLVANIA) 01 May 2014 (2014-05-01)<br>entire document | 1-20 |

Form PCT/ISA/210 (second sheet) (January 2015)

**INTERNATIONAL SEARCH REPORT**
Information on patent family members

International application No.

**PCT/CN2020/102564**

| Patent document cited in search report | | | Publication date (day/month/year) | Patent family member(s) | | | Publication date (day/month/year) |
|---|---|---|---|---|---|---|---|
| CN | 106190774 | A | 07 December 2016 | None | | | |
| CN | 107402295 | A | 28 November 2017 | CN | 107402295 | B | 30 August 2019 |
| CN | 204939452 | U | 06 January 2016 | None | | | |
| CN | 107723207 | A | 23 February 2018 | CN | 107723207 | B | 01 January 2019 |
| | | | | WO | 2019085388 | A1 | 09 May 2019 |
| | | | | US | 2020238286 | A1 | 30 July 2020 |
| CN | 103834558 | A | 04 June 2014 | None | | | |
| CN | 109852530 | A | 07 June 2019 | None | | | |
| CN | 208604119 | U | 15 March 2019 | None | | | |
| CN | 109852544 | A | 07 June 2019 | None | | | |
| WO | 2014065861 | A1 | 01 May 2014 | US | 9846157 | B2 | 19 December 2017 |
| | | | | US | 2018113130 | A1 | 26 April 2018 |
| | | | | US | 2015285786 | A1 | 08 October 2015 |
| | | | | US | 10338071 | B2 | 02 July 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910666239